(19)

Europäisches
Patentamt
European
Patent Office
Office européen
des brevets

(11) **EP 2 120 691 B1**

(12) **EUROPÄISCHE PATENTSCHRIFT**

(45) Veröffentlichungstag und Bekanntmachung des Hinweises auf die Patenterteilung:
**29.12.2010 Patentblatt 2010/52**

(21) Anmeldenummer: **08700303.4**

(22) Anmeldetag: **05.02.2008**

(51) Int Cl.:
***A61B 5/04*** *(2006.01)*

(86) Internationale Anmeldenummer:
**PCT/AT2008/000037**

(87) Internationale Veröffentlichungsnummer:
**WO 2008/095213 (14.08.2008 Gazette 2008/33)**

(54) **VERFAHREN ZUR BESTIMMUNG DER TRAININGSINTENSITÄT**

METHOD FOR DETERMINING THE INTENSITY OF A WORKOUT

PROCEDE DE DETERMINATION DE L'INTENSITE D'ENTRAINEMENT

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **06.02.2007 AT 1952007**

(43) Veröffentlichungstag der Anmeldung:
**25.11.2009 Patentblatt 2009/48**

(73) Patentinhaber: **Greiner Bio-One International AG
4550 Kremsmünster (AT)**

(72) Erfinder: **KLEPP, Nikolaus
A-4810 Gmunden (AT)**

(74) Vertreter: **Ofner, Clemens et al
Anwälte Burger & Partner
Rechtsanwalt GmbH
Rosenauerweg 16
4580 Windischgarsten (AT)**

(56) Entgegenhaltungen:
**EP-A- 1 127 543     DE-A1-102004 052 083**

- **MICHAELM BRENNAN ET AL: "Do Existing Measures of Poincaré Plot Geometry Reflect Nonlinear Features of Heart Rate Variability?" IEEE TRANSACTIONS ON BIOMEDICAL ENGINEERING, IEEE SERVICE CENTER, PISCATAWAY, NJ, US, Bd. 48, Nr. 11, 1. November 2001 (2001-11-01), XP011007162 ISSN: 0018-9294**

**Beschreibung**

Die Erfindung bezieht sich auf ein Verfahren und eine Vorrichtung zur Bestimmung der Belastungsintensität bzw. der Trainingsintensität einer Person, wobei die Herzfrequenzinformation der Person gemessen wird, entsprechend den Merkmalen in den Oberbegriffen der Ansprüche 1, 2 und 19.

Insbesondere für sportmedizinische Anwendungen ist die Frage einer optimalen Trainings- bzw. Belastungsintensität einer trainierenden Person von zentraler Bedeutung. Reine Herzfrequenz-Leistungstests sind hierzu jedoch völlig ungeeignet, da die Herzfrequenz annähernd proportional zur Belastungsintensität ist und sich daraus ein optimales Trainingsniveau nicht bestimmen lässt. Dieses ist individuell verschieden und lässt sich nur über die Laktatkonzentration im Blut bestimmen. Laktat, das Endprodukt des anaeroben laktaziden Stoffwechsel, ist der beste Indikator für eine objektive Beurteilung der Ausdauerleistungsfähigkeit. Es wird gebildet, wenn während einer intensiven Ausdauerbelastung der über die Atmung aufgenommene Sauerstoff nicht ganz ausreicht, um den im Muskel benötigten Energiebedarf zur Muskelkontraktion zu decken. Unter Belastung wird ständig Laktat gebildet, aber auch wieder eliminiert, sodass bei steigender Intensität eine Laktatkonzentration erreicht wird, bei der die Bildung gerade noch der Elimination entspricht. Dieser Bereich ist in der Sportmedizin als maximales Laktat-Steady-State (maxlass), als aerobe/anaerobe Schwelle oder als Fettstoffwechselgrenze bekannt. Eine zuverlässige Beurteilung und Steuerung der Ausdauerleistungsfähigkeit ist also nur mit Hilfe der Kenntnis der Blutlaktatkonzentration bei unterschiedlicher körperlicher Belastung, d.h. nur in Kenntnis der individuellen Stoffwechselsituation, möglich. Testverfahren, die nur die Herzfrequenz in Beziehung zur Leistung setzen, sind zur Bestimmung des optimalen Trainingsniveaus nicht geeignet.

Um den Verlauf der individuellen Blutlaktatkonzentration bei unterschiedlichen Belastungen bestimmen zu können, werden allerdings aufwendige Untersuchungen mit begleitend vorgenommen Blutabnahmen und Analysen erforderlich. Insbesondere für begleitende Messungen während des Trainings sind derartige Messungen aber völlig unpraktikabel. Es wurden daher Versuche unternommen, Verfahren zu entwickeln, die eine nicht invasive Abschätzung des Laktatgehalts im Körper im Zusammenhang mit körperlicher Anstrengung ermöglichen. Ein derartiges Verfahren ist beispielsweise in dem Dokument EP 1 127 543 B1 beschrieben. Dabei werden Herzfrequenzinformationen einer Person gemeinsam mit einem die Physiologie der Person darstellenden Parameter in ein mathematisches Modell eingegeben, das die Laktatkonzentration im Körper modelliert und so einen Schätzwert der Laktatkonzentration in dem Körper der trainierenden Person liefert. Es zeigt sich jedoch, dass die verwendeten physiologischen Parameter, wie Alter, Gewicht, Größe und Geschlecht als auch die Eingabe von Stressparametern, wie die Geschwindigkeit oder der Widerstand eines Übungsfahrrads, nicht nur unzureichend sind, um ein zuverlässiges Ergebnis zu erhalten, sondern dass dies auch einer nutzerfreundlichen Anwendung im Breitensport hinderlich ist.

Die Aufgabe der vorliegenden Erfindung ist es daher, ein Verfahren bzw. eine Vorrichtung zur Bestimmung der Trainingsintensität einer Person zu schaffen, das es ermöglicht, auf der Basis von Messungen der Herzfrequenz die optimale Trainingsintensität einer Person feststellen zu können.

Die Aufgabe der Erfindung wird durch das Verfahren entsprechend den Verfahrensschritten des Anspruchs 1 gelöst. Dem gemäß ist bei dem Verfahren zur Bestimmung der Trainingsintensität einer Person die Messung der Herzfrequenzinformation vorgesehen, wobei zunächst die zeitlichen Intervalle $RR_i$ zwischen jeweils aufeinander folgenden Herzschlägen aufgezeichnet werden. Durch Festlegen einer Anzahl N von aufeinander folgenden Intervallen $RR_i$ oder durch Festlegen eines Beobachtungsintervalls wird eine Folge von Intervallen $RR_i$ ausgewählt und aus der Folge dieser Intervalle $RR_i$ durch eine mathematische Abbildung eine Folge von normierten Intervallen $rr_i$ erzeugt. Sodann werden zu jeweils zwei aufeinander folgenden normierten Intervallen $rr_i$ und $rr_{i+1}$ Punkte in einem Poincare-Plot der normierten Intervalle $rr_i$ erzeugt und durch eine orthogonale Regressionsanalyse eine Hauptlängenachse $X_0$ und eine Hauptbreitenachse $Y_0$ der Punkte der Folge der normierten Intervalle $rr_i$ in Poincare-Plot bestimmt. Aus den orthogonalen Abständen dieser Punkte bezüglich der Hauptlängenachse $X_0$ wird sodann die Standardabweichung $SO_L$ berechnet. Als Ergebnis dieser Teilanalyse wird ein Wertepaar, bestehend aus einem Mittelwert $RR_{MW}$ der Folge der Intervalle $RR_i$ und aus der Standardabweichung $SO_L$ abgespeichert. Die vorgenannten Analyse-Schritte werden für weitere Folgen von Intervallen $RR_i$ fortgesetzt. Aus den so ermittelten Wertepaaren von den Mittelwerten $RR_{MW}$ entsprechenden Herzfrequenzen HF ($HF = 1 / RR_{MW}$) und den Standardabweichungen $SO_L$ wird sodann eine Trainingsintensitätskurve der Person erzeugt. Durch Vergleich der zuletzt bestimmten Herzfrequenz HF mit der ermittelten Trainingsintensitätskurve wird schließlich die momentane, spezifische Trainingsintensität der Person ermittelt. Vorteilhaft an diesen Verfahren ist, dass damit trainingsbegleitende Überwachungen und die Feststellung des optimalen Belastungsbereiches einer trainierenden Person ohne vorherige aufwendige Untersuchungen, wie beispielsweise Blutabnahmen und Blutlaktatkonzentrationsbestimmungen möglich sind.

Die Aufgabe der Erfindung wird eigenständig auch durch das Verfahren entsprechend den Verfahrensschritten des Anspruchs 2 gelöst. Dabei wird, zusätzlich zum vorstehend beschriebenen Verfahren, neben der Standardabweichung $SO_L$ aus orthogonalen Abständen der Punkte der Folge der normierten Intervalle $rr_i$ bezüglich der Hauptbreitenachse $Y_0$ auch noch eine Standardabweichung $SO_W$ berechnet. Als Ergebnis der Teilanalysen werden Wertepaare, bestehend aus einem Mittelwert $RR_{MW}$ der Folge der Intervalle $RR_i$ und einem aus der Standardabweichung $SO_L$ und der Standardabweichung $SO_W$ gebildeten Quotienten s = $SO_L$ / $SO_W$ abgespeichert. Aus den so ermittelten Wertepaaren wird sodann eine Trainingsintensitätskurve der Person erzeugt und durch Vergleich der zuletzt bestimmten Herzfrequenz HF mit der ermittelten Trainingsintensitätskurve die momentane, spezifische Trainingsintensität bzw. der physische Belastungszustand der Person ermittelt.

Gemäß einer Weiterbildung des Verfahrens ist vorgesehen, dass die zur Analyse auszuwählenden Folgen der Intervalle $RR_i$ aus einander zeitlich überlappenden Beobachtungsintervallen ausgewählt werden. Dies hat den Vorteil, dass relativ rasch eine ausreichend hohe Anzahl von Wertepaaren zur Bestimmung der Trainingsintensitätskurve erhalten wird und somit rascher und zuverlässiger Aussagen über die momentane Trainingsintensität gefolgert werden können.

Von Vorteil sind auch die Weiterbildungen des Verfahrens gemäß den Ansprüchen 6 bis 9, da damit die Zuverlässigkeit der Auswerteergebnisse verbessert werden kann.

Die Ausführungsvarianten entsprechend den Ansprüchen 10 bis 15 haben den Vorteil, dass damit Trainingsintensitätskurven mit stärker bzw. deutlicher hervortretenden Kurvenabschnitten sowohl im unteren als auch im oberen Belastungsbereich erhalten werden können. Der optimale Trainingsbereich kann damit zuverlässiger festgelegt werden.

Die Weiterbildung des Verfahrens gemäß Anspruch 16 hat den Vorteil, dass durch die verwendete Approximation der Trainingsintensitätskurve eine erfahrungsgemäß ausreichend gute Näherung zur Verfügung steht.

Vorteilhaft ist auch die Weiterbildung des Verfahrens entsprechend Anspruch 17, da damit eine zeitlich fortschreitende, immer stabiler werdende Trainingsintensitätskurve und eine zuverlässigere Überwachung der Trainings- bzw. Belastungsintensität einhergeht.

Gemäß einer Weiterbildung des Verfahrens ist vorgesehen, dass die Trainingsintensitätskurve nach Abschluss des Trainings gemeinsam mit Daten, wie dem Namen der Person und den Daten des durchgeführten Trainings, gespeichert werden. Dies hat den Vorteil, dass spätere Vergleiche ermöglicht werden und Rückschlüsse über längerfristige Entwicklungen der Leistungsfähigkeit einer Person angestellt werden können.

Die Aufgabe der Erfindung wird eigenständig auch durch die Vorrichtung zur Überwachung der Belastungsintensität einer Person gemäß Anspruch 19 gelöst. Dem nach ist vorgesehen, dass die Vorrichtung eine am Körper befestigbare Einheit mit einem Sensor zur Aufzeichnung von Herzschlägen umfasst, wobei die Vorrichtung eine Auswerteeinheit zur Durchführung eines Verfahrens, wie vorstehend beschrieben, und eine Ausgabeeinheit zur Information der Person über die ermittelte Belastungsintensität umfasst.

Die Erfindung wird im nachfolgenden anhand der in den Zeichnungen dargestellten Ausführungsbeispiele näher erläutert.

Es zeigen:

Fig. 1    eine Vorrichtung, die zur Überwachung der Belastungsintensität an einer trainie- renden Person befestigt ist;

Fig. 2    eine Prinzipdarstellung des Brustgurts und der Überwachungseinheit der Vorrich- tung gemäß Fig.1;

Fig. 3    ein Diagramm mit den Werten der zeitlichen Intervalle $RR_i$ von aufeinander fol- genden Herzschlägen;

Fig. 4    ein Diagramm einer Folge von normierten Intervallen $rr_i$;

Fig. 5    einen Poincaré-Plot der normierten Intervalle $rr_i$;

Fig. 6    ein Diagramm der Standardabweichungen $SO_L$ bei unterschiedlichen Belastungs- intensitäten bzw. unterschiedlichen Herzfrequenzen HF;

Fig. 7    ein alternatives Ausführungsbeispiel einer Trainingsintensitätskurve;

Fig. 8    ein Diagramm von Intervallen $RR_i$ mit einem Artefakt.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

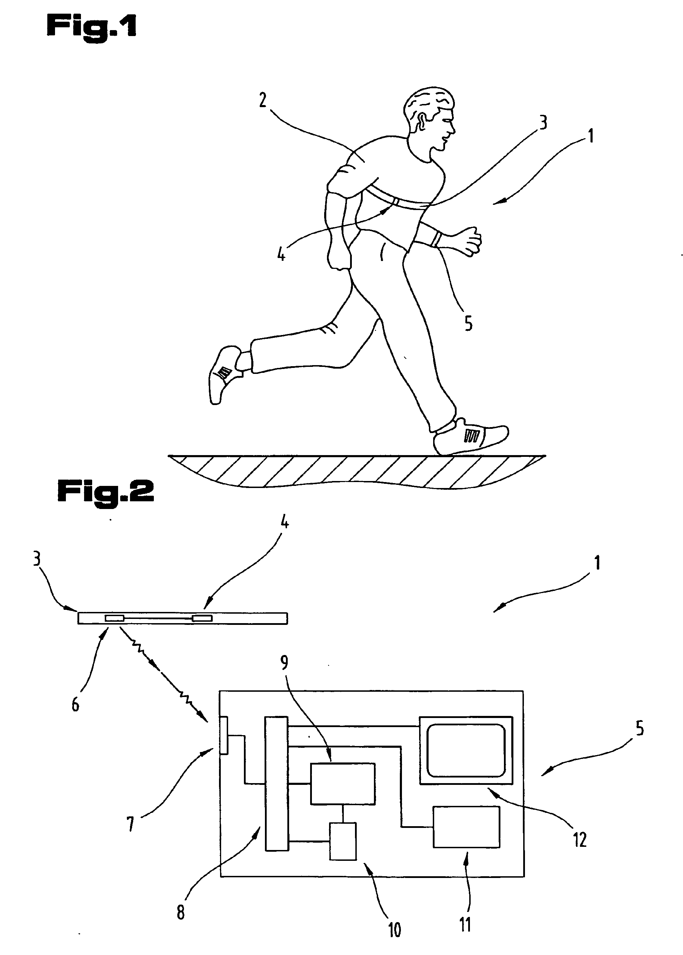

Die Fig. 1 zeigt eine Vorrichtung 1, die zur Überwachung der Belastungsintensität an einer trainierenden Person 2 befestigt ist.

Gemäß diesem Ausführungsbeispiel umfasst die Vorrichtung 1 einen Brustgurt 3, der mit einem Sensor 4 zur Aufzeichnungen von Herzschlägen ausgestattet ist und einer Überwachungseinheit 5, die ähnlich einer Armbanduhr von der Person 2 getragen wird.

Der Sensor 4 weist Elektroden auf, sodass in an sich bekannter Weise ein Elektrokardiogramm (EKG) der Herzaktivitäten der Person 2 aufgezeichnet werden kann.

In der Fig. 2 ist eine vereinfachte Prinzipdarstellung des Brustgurts 3 und der Überwachungseinheit 5 der Vorrichtung 1 wiedergegeben.

Neben dem Sensor 4 weist der Brustgurt 3 auch einen Sender 6 für die Übertragung der detektierten Signale zu der Überwachungseinheit 5 auf. Diese wiederum verfügt über einen Empfänger 7 als Signaleingang für die Weiterverarbeitung der Herzfrequenzinformation. Die Überwachungseinheit umfasst weiters eine zentrale Steuerung 8, eine Auswerteeinheit 9 und einen Speicher 10 zur Ablage bzw. zum Zwischenspeichern der aufgezeichneten Herzfrequenzinformationen bzw. der infolge der Auswertung berechneten Daten. Zur Bedienung der Überwachungseinheit 5 ist eine Bedieneinheit 11 und zur Darstellung der Ergebnisse der Datenauswertung eine Ausgabe bzw. Anzeige 12 vorgesehen. Zur Durchführung des erfindungsgemäßen Verfahrens ist in der Auswerteeinheit 9 bzw. der zentralen Steuerung 8 ein Computerprogramm geladen.

In der Auswerteeinheit 9 werden aus den Signalen der Herzaktivität der Person 2 die zeitlichen Abstände zwischen jeweils-aufeinander folgenden Herzschlägen gemessen und in dem Speicher 10 abgelegt.

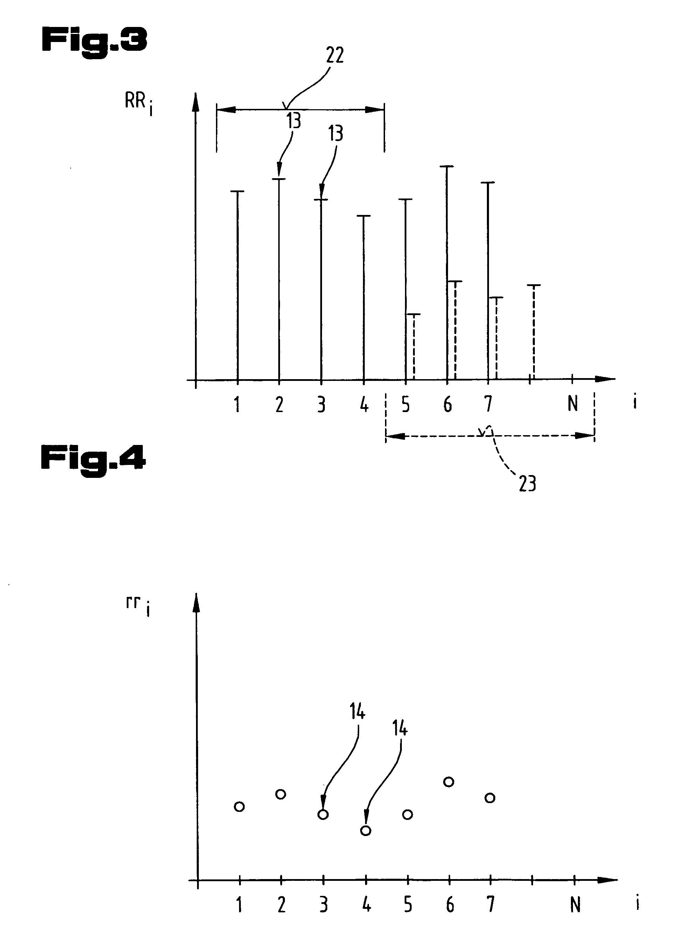

Anhand der folgenden Fig. 3 bis 6 wird nun ein erstes Ausführungsbeispiel des Verfahrens beschrieben.

Die Fig. 3 zeigt ein Diagramm mit Intervallen $RR_i$ 13 von aufeinander folgenden Herzschlägen.

Die zeitlichen Abstände zwischen den einzelnen Herzschlägen sind dabei durch die auf der Abszisse aufgetragene Nummer i und das auf der Koordinate aufgetragene Intervall $RR_i$ 13 repräsentiert. Die Intervalle $RR_i$ 13 werden üblicherweise in ms (Millisekunden), das heißt, als zeitliche Dauer zwischen zwei aufeinander folgenden Herzschlägen ausgedrückt. Alternativ ist es auch möglich, anstelle der Nummer i den momentanen Zeitpunkt eines Herzschlags als die auf der Abszisse aufzutragende Größe zu verwenden. Im nächsten Verfahrensschritt werden aus den fortlaufend aufgezeichneten Intervallen $RR_i$ 13 Folgen von Intervallen $RR_i$ 13 ausgewählt, die als Grundlage für Analysen der Variabilität der Herzfrequenz HF dienen. Die Analyse erfolgt in diskreten Einheiten, beispielsweise einer festen Anzahl von 150 Intervallen $RR_i$ 13 oder aller innerhalb eines Beobachtungsintervalls von beispielsweise 1,5 Minuten registrierten Herzschläge. Eine solche Folge von Intervallen $RR_i$ 13 wird also beispielsweise gebildet durch eine Anzahl von N = 150 zeitlich aufeinander folgenden Intervallen $RR_i$ 13 (i = 1... N).

Aus der Folge von Intervallen $RR_i$ 13 wird in einem nächsten Verfahrensschritt eine Folge von normierten Intervallen $rr_i$ 14 erzeugt, wie in Fig. 4 dargestellt ist. Gemäß einem ersten Ausführungsbeispiels des Verfahrens wird die Folge der normierten Intervalle $rr_i$ 14 dadurch gebildet, dass jedes Intervall $RR_i$ 13 durch einen Mittelwert $RR_{MW}$ der Folge der Intervalle $RR_i$ 13 dividiert wird.

$$rr_i = RR_i\ /\ RR_{MW};\ i = 1\ ...\ N$$

Während die Intervalle $RR_i$ 13 die Dimension einer Zeit haben (üblicherweise ausgedrückt in ms) handelt es sich bei den normierten Intervallen $rr_i$ 14 somit um dimensionslose Größen.

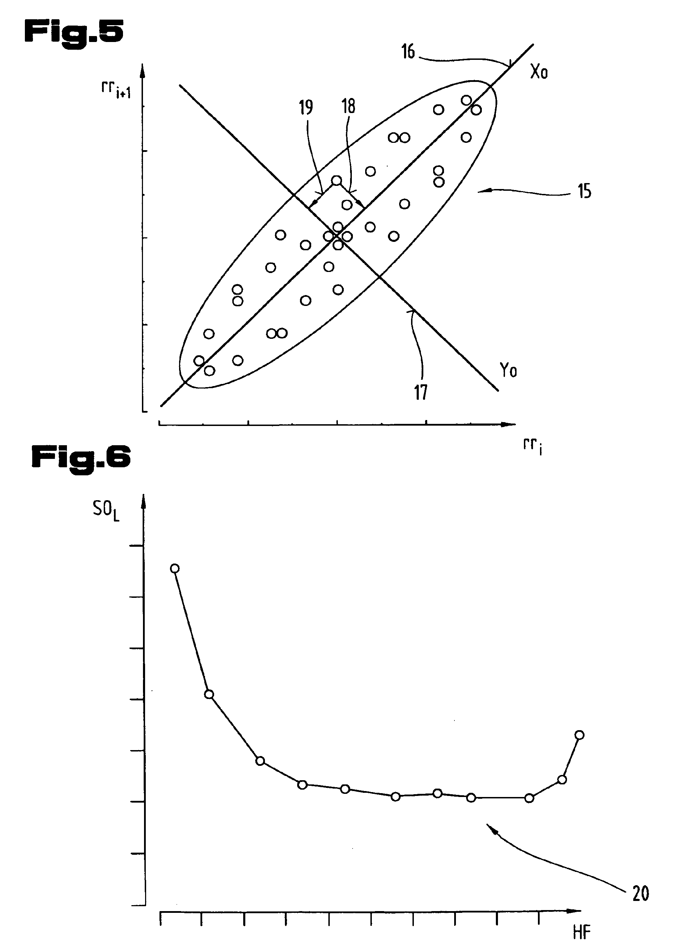

Die Fig. 5 zeigt einen Poincaré-Plot der normierten Intervalle $rr_i$ 14.

Dazu werden Wertepaare aus aufeinander folgenden normierten Intervallen $rr_i$ 14 und $rr_{i+1}$ als Punkte in das Diagramm gemäß Fig. 5 eingetragen. In dieser zweidimensionalen grafischen Darstellung ergibt sich somit eine Punktwolke 15, in der die Variabilität der Herzfrequenz HF in einer Weise zum Ausdruck kommt, die eine weitergehende quantitative Analyse erlaubt. Der Poincare-Plot der normierten Intervalle $rr_i$ 14 kann auch als eine Visualisierung der Tatsache gesehen werden, dass sich aufeinander folgende Herzschläge in ihrer Dauer, d.h. der Länge der Intervalle $RR_i$ 13 stets unterscheiden. In einem nächsten Verfahrensschritt werden, wiederum durch das Computerprogramm in der Überwachungseinheit 5, zu den Punkten der Punktwolke 15 in dem Poincaré-Plot eine Hauptlängenachse $X_0$ 16 und eine Hauptbreitenachse $Y_0$ 17 einer so genannten Vertrauensellipse, insbesondere einer 95 % Vertrauensellipse, berechnet. Dies kann mittels Methoden der Regressionsanalyse bzw. orthogonaler Regressionsanalyse erfolgen. Bezüglich der Hauptlängenachse $X_0$ 16 weist somit jeder Punkt der Punktwolke 15 eine orthogonalen Abstand 18 und bezüglich der Hauptbreitenachse $Y_0$ 17 einen orthogonalen Abstand 19 auf. In einem weiteren Verfahrensschritt wird nun aus den orthogonalen Abständen 18 eine Standardabweichung $SO_L$ berechnet. Diese Standardabweichung $SO_L$ der orthogonalen Abstände 18 bezüglich der Hauptlängsachse $X_0$ 16 wird gemeinsam mit dem Mittelwert $RR_{MW}$ der Folge der Intervalle $RR_i$ 13 als Ergebnis der Auswertung der Folge der Intervalle $RR_i$ 13 abgespeichert.

Die vorstehend beschriebenen Verfahrensschritte werden fortlaufend für weitere Folgen von Intervallen $RR_i$

13 durchgeführt und jeweils die entsprechenden Werte der Standardabweichung $SO_L$ und des Mittelwerts $RR_{MW}$ der Folge der Intervalle $RR_i$ 13 aufgezeichnet bzw. abgespeichert. Diese Wertepaare können in einem zweidimensionalen Diagramm dargestellt werden, wobei auf der Abszisse die dem Mittelwert $RR_{MW}$ der jeweiligen Folgen der Intervalle $RR_i$ 13 entsprechende Herzfrequenz HF (d.h. der Kehrwert HF = 1/ $RR_{MW}$) und auf der Ordinate die jeweilige Standardabweichung $SO_L$ aufgetragen wird (Fig. 6).

Die Fig. 6 zeigt ein Diagramm der Standardabweichungen $SO_L$ bei unterschiedlichen Belastungsintensitäten.

Die gemäß dem vorstehend beschriebenen Verfahren bestimmten Wertepaare aus den Mittelwerten $RR_{MW}$ und den jeweiligen Standardabweichungen $SO_L$ bestimmen eine für die jeweilige Person 2 charakteristische Belastungsintensitätskurve bzw. Trainingsintensitätskurve 20. Da bekanntlich die Herzfrequenz HF proportional ist zur Leistung bzw. Belastungsintensität können die auf der Abszisse aufgetragenen Werte der Herzfrequenz HF als gleichbedeutend der jeweiligen Belastungsintensität angesehen werden. Die Trainingsintensitätskurve 20, wie sie durch die aus dem vorstehend beschriebenen Verfahren hervorgegangenen Wertepaare von Mittelwerten $RR_{MW}$ und Standardabweichungen $SO_L$ bestimmt wird, zeigt charakteristische Eigenschaften, die zur Beurteilung der momentanen Belastung bzw. Trainingsintensität der Person 2 von großem Nutzen sind. Ausgehend von einem Minimum in der Trainingsintensitätskurve 20 weisen die Standardabweichungen $SO_L$ hin zu niedrigen Belastungen bzw. hin zu niedrigen Herzfrequenzen HF aber auch hin zu höheren Belastungen bzw. hin zu höheren Herzfrequenzen HF einen Anstieg auf. Untersuchungen zeigen, dass das Minimum der Standardabweichung einem Wert von etwa 70 % der Leistung des Laktat-Steady-State entspricht und dabei Laktatwerte unterhalb von 2 mmol/l, wie es für ein gesundheitsorientiertes Ausdauertraining empfehlenswert ist, erreicht werden.

Die Trainingsintensitätskurve 20 ermöglicht schließlich die Ermittlung der momentanen spezifischen Trainingsintensität der Person 2, in dem die zuletzt bestimmten Werte, d.h. der Mittelwert $RR_{MW}$ der Folge der Intervalle $RR_i$ 13 und die entsprechende Standardabweichung $SO_L$ mit dem Verlauf der Trainingsintensitätskurve 20 verglichen werden. Dabei ist zu beachten, dass die Trainingsintensitätskurve 20 bei der erstmaligen Benutzung der Vorrichtung durch die Person 2 noch nicht vorhanden ist und sich erst allmählich im Verlauf des fortschreitenden Trainings und der damit einher gehenden, durch die Überwachungseinheit 5 erfolgenden Auswertungen der Herzfrequenzinformationen der Person 2 durch Abspeichern der entsprechenden Wertepaare, bestehend aus den Mittelwerten $RR_{MW}$ der Folge der Intervalle $RR_i$ 13 und den entsprechenden Standardabweichungen $SO_L$ ergibt. Die praktische Anwendung der Vorrichtung 1 durch die Person 2 wird beispielsweise so ablaufen, dass die Person 2 zunächst über die Bedieneinheit 11 die Überwachungseinheit 5 in Betrieb nimmt und sodann mit steigender Belastung zu trainierend beginnt. Mit fortschreitender Dauer des Trainings werden somit allmählich Punkte der Trainingsintensitätskurve 20 in deren abfallenden Ast bzw. im unteren Belastungsbereich berechnet und aufgezeichnet. Erwartungsgemäß wird die Person 2 im Verlauf des fortlaufenden Trainings die Belastung allmählich steigern, sodass auch Punkte der Trainingsintensitätskurve 20 aus dem oberen Belastungsbereich berechnet werden können. Es ergibt sich somit alleine aus den wechselnden Belastungen der trainierenden Person 2 bei fortlaufendem Training eine Vervollständigung der Trainingsintensitätskurve 20.

Erfindungsgemäß ist vorgesehen, dass der Person 2 durch die Überwachungseinheit 5 das Ergebnis des Vergleichs der momentanen Werte der Standardabweichung $SO_L$ bzw. der Herzfrequenz HF entsprechend dem Mittelwert $RR_{MW}$ mit der Trainingsintensitätskurve 20 mitgeteilt wird. Dies kann beispielsweise dadurch erfolgen, dass auf der Anzeige 12 der Überwachungseinheit 5 eine grafische Darstellung entsprechend jener in Fig. 6 wiedergegeben wird, wobei die zuletzt bestimmten Werte besonders hervorgehoben sind. Die Person 2 hat damit die Möglichkeit ihre momentane Belastung in Verhältnis zu ihrer eigenen Trainingsintensitätskurve 20 abzulesen und in Reaktion darauf diese bedarfsweise zu erhöhen, zu verringern oder gleich zu belassen. Alternativ dazu könnte auch vorgesehen werden, dass die Überwachungseinheit 5 über eine akustische Ausgabe verfügt, über die die Person 2 entsprechende Informationen ihrer momentanen spezifischen Trainingsintensität erhält.

Zur Durchführung der verfahrensgemäß vorgesehenen Analysen der Herzfrequenzinformationen bzw. Auswertungen der Daten sind in der Überwachungseinheit 5 bzw. dem Computer-programm der Auswerteeinheit 9 entsprechende Algorithmen vorgesehen. Dabei erfolgt die Bestimmung des individuellen Minimums der Standardabweichung $SO_L$ regressionsanalytisch in dem nach der Methode der Minimierung der Abweichungsquadrate ein Polynom $SO_L(HF)$, bevorzugt ein Polynom dritten Grades, durch die Punkte der Trainingsintensitätskurve 20 gelegt wird. D.h. die Punkte der Trainingsintensitätskurve 20 werden durch das Polynom angenähert.

$$SO_L(HF) = s_0 + s_1\, HF + s_2\, HF^2 + s_3\, HF^3$$

Das Minimum der Standardabweichung $SO_L$ wird sodann, ausgehend von dem regressionsanalytisch bestimmten Polynom $SO_L(HF)$ differenzialanalytisch bestimmt. Bei der Durchführung des Verfahrens ist weiters vorgesehen, dass die derart berechnete Trainingsintensitätskurve 20 bzw. das Polynom $SO_L(HF)$ in regelmäßigen Abständen neu berechnet wird. Vorzugsweise wird nach jeder Bestimmung eines neuen Wertepaars der zuletzt analysierten Folge von Intervallen RR$_i$ 13, d.h. dem zuletzt bestimmten Mittelwert RR$_{MW}$ der Folge der Intervalle RR$_i$ 13 und der zuletzt bestimmten entsprechenden Standardabweichung SO$_L$ eine Neuberechnung des die Trainingsintensitätskurve 20 darstellenden Polynoms durchgeführt. Dies hat zur Folge, dass durch die allmähliche Vergrößerung der Anzahl der Punkte in den dem Diagramm gemäß Fig. 6 der Verlauf der Trainingsintensitätskurve 20 allmählich immer stabiler und somit die Überwachung der Trainings- bzw. Belastungsintensität immer verlässlicher wird.

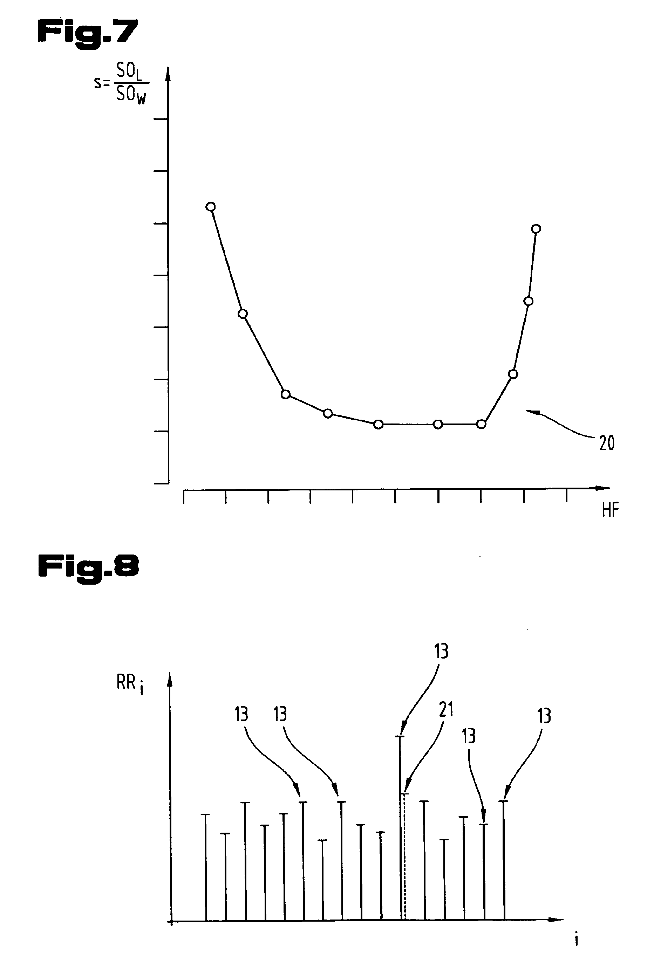

Anhand der Fig. 7 wird nun ein alternatives Beispiel für eine Trainingsintensitätskurve 20 beschrieben. Entsprechend der Darstellung der Punkte der Punktwolke 15 im Poincare- Plot gemäß Fig. 5 wird dazu neben der Standardabweichung SO$_L$ aus den orthogonalen Abständen 19 bzgl. der Hauptbreitenachse Y$_0$ 17 eine Standardabweichung SO$_W$ berechnet. Aus den beiden Standardabweichungen SO$_L$ und SO$_W$ wird nun durch Quotientenbildung eine neue charakteristische Größe, und zwar ein Quotient s = SO$_L$ / SO$_W$ gebildet. Als Ergebnis der Auswertung einer Folge von Intervallen RR$_i$ 13 wird nun das Wertepaar aus dem Quotienten s und dem Mittelwert RR$_{MW}$ abgespeichert. Diese Verfahrensschritte werden fortlaufend für weitere Folgen von Intervallen RR$_i$ 13 durchgeführt und die dabei erhaltenen Werte der Quotienten s = SO$_L$ / SO$_W$ und des Mittelwerts RR$_{MW}$ der jeweiligen Folgen der Intervallen RR$_i$ 13 aufgezeichnet. Diese Wertepaare können nun ebenfalls, wie vorstehend im Bezug auf die Standardabweichung SO$_L$ beschrieben worden ist, in einem zweidimensionalen Diagramm dargestellt werden. Im Diagramm der Fig. 7 ist auf der Abszisse die den Mittelwert RR$_{MW}$ der jeweiligen Folgen der Intervalle RR$_i$ 13 entsprechende Herzfrequenz HF und auf der Ordinate die jeweilige charakteristische Größe s = SO$_L$ / SO$_W$ aufgetragen und erhält man so eine alternative Trainingsintensitätskurve 20. Die Punkte dieser Trainingsintensitätskurve 20 können wiederum durch ein Polynom angenähert werden.

$$s(HF) = u_0 + u_1\,HF + u_2\,HF^2 + u_3\,HF^3$$

Die erhaltene Trainingsintensitätskurve 20 gemäß Fig. 7 erlaubt nun ebenfalls, die Überwachung der momentanen Trainings- bzw. Belastungsintensität einer unter wechselnder physischer Belastung stehenden Person 2.

Es ist zu betonen, dass bei dem beschriebenen Verfahren mit der Messung der Intervalle RR$_i$ 13 das Auslangen gefunden werden kann. Eine vorherige Untersuchung der Person 2 zur Feststellung ihrer individuellen Leistungsmerkmale ist nicht notwendig und kann darauf verzichtet werden, da sich diese personenspezifischen Merkmale aus der vorstehend beschriebenen Ermittlung der Trainingsintensitätskurve 20 entsprechend der aktuellen Leistungsfähigkeit bzw. Tagesverfassung der Person 2 automatisch ergeben. Eine Voraufzeichnung einer Trainingsintensitätskurve 20 für eine spätere Verwendung zur Beurteilung der momentanen Trainingsintensität ist nicht nur nicht erforderlich, sondern wäre sogar nachteilig, da diese in Folge einer veränderten Tagesverfassung oder eines veränderten Leistungsniveaus der Person 2 sogar zu einer falschen Beurteilung führen könnte. Gemäß dem Verfahren ist im Gegensatz dazu sogar vorgesehen, dass nach Abschluss eines Trainings der Person 2 die dabei erhaltene Trainingsintensitätskurve 20 durch entsprechende Eingaben an der Bedieneinheit 11 der Überwachungseinheit 5, ergänzt durch Daten, wie dem Namen der Person 2 und den Daten des vorgenommenen Trainings, in den Speicher 10 der Überwachungseinheit 5 abgespeichert wird. Dies erlaubt es aus später durchgeführten Vergleichen der Trainingsintensitätskurven 20 von in zeitlich größeren Abständen durchgeführten Trainings Rückschlüsse über die langfristige Entwicklung der physischen Leistungsfähigkeit der Person zu ziehen.

Gemäß einer Ausführungsvariante des erfindungsgemäßen Verfahrens erfolgt, der vorstehend beschriebenen Analyse und Auswertung der Herzfrequenzinformationen vorausgehend, eine Datenaufbereitung. In einer ersten Stufe der Datenaufbereitung der Intervalle RR$_i$ 13 werden Artefakte identifiziert und eliminiert. Dazu wird aus den Intervallen RR$_i$ 13 ein gleitender Referenzwert 21 berechnet und festgelegt, dass ein Intervall RR$_i$ 13 dann als Artefakt anzusehen ist, wenn dieser mehr als ein vorwählbarer Prozentsatz, beispielsweise mehr als 30 %, von dem zuvor berechneten Referenzwert 21 abweicht. Als Referenzwert 21 zu einem Intervall RR$_i$ 13 wird dabei diesem der Median der fünf vorhergehend und fünf nachfolgenden Intervalle RR$_i$ 13 zugeordnet. Zu Berechnung des Referenzwerts 21 sollten zumindest eine Anzahl von zumindest drei vorhergehenden und zumindest drei nachfolgenden Intervallen RR$_i$ 13 verwendet werden. Trifft nun für ein Intervall RR$_i$ 13 die Bedingung zu, dass dieses mehr als bevorzugt 30 % von dem ihm zugeordneten Referenzwert 21 abweicht, so wird der Wert des betreffende Intervalls RR$_i$ 13 durch das jeweilige Referenzintervall 21 ersetzt (Fig. 8). Die Fig. 8 zeigt eine Folge von Intervallen RR$_i$ 13 entsprechend Fig. 3, wobei das Eliminieren eines Artefakts beispielhaft dargestellt ist.

In einer zweiten Stufe der Datenaufbereitung ist in dem Verfahren eine Auswahl der Beobachtungsintervalle bzw. der Folgen von Intervallen RR$_i$ 13 zur weiteren Auswertung vorgesehen. Dazu wird eine obere Grenze, beispielsweise ein vorwählbarer Prozentsatz für die Artefakthäufigkeit von beispielsweise 5 % bestimmt und festgelegt, dass Beobachtungsintervalle bzw. Messabschnitte, die eine darüber hinaus gehende Artefakthäufigkeit aufweisen, von weiteren Auswertungen ausgeschlossen werden. Die vorgenannten Maßnahmen der Datenaufbereitung ermöglichen eine Verbesserung der Zuverlässigkeit der Analyseergebnisse bzw. der Berechnung der Trainingsintensitätskurve 20.

Anstelle der Auswahl von zeitlich aufeinander folgenden Beobachtungsintervallen bzw. der Auswahl von zeitlich aufeinander folgenden Folgen von Intervallen $RR_i$ 13 ist es auch möglich, dass zeitlich einander überlappende Beobachtungsintervalle zur Analyse und weiteren Auswertung bzw. Berechnung der Trainingsintensitätskurve 20 herangezogen werden. Dies hat den Vorteil, dass in kürzerer Zeit eine ausreichend hohe Anzahl von Punkten der Trainingsintensitätskurve 20 in der Darstellung gemäß Fig. 6 erhaltern werden und somit rascher zuverlässige Aussagen über die momentane Trainingsintensität aus dem Verfahren gefolgert werden können.

Als vorteilhaft erweist sich auch, bei der Auswahl der Folge von Intervallen $RR_i$ 13 besondere Ereignisse in dem Trainingsverlauf zu berücksichtigen. Ein solches besonderes Ereignis bildet beispielsweise eine sprunghafte Änderung der Herzfrequenz HF, wie es in Fig. 3 durch die strichliert dargestellten Intervalle $RR_i$ (für i = 5, 6 usw.) dargestellt ist. Es erweist sich als günstig, die Beobachtungsintervalle für die Auswahl der Folgen von Intervallen $RR_i$ 13 so zu wählen, dass solche sprunghaften Änderungen der Herzfrequenz HF darin nicht enthalten sind. So reicht beispielsweise ein erstes Beobachtungsintervall 22 (Fig. 3) bis kurz vor die Stelle der sprunghaften Veränderung der Herzfrequenz HF. Während dessen ein darauf folgendes, weiteres Beobachtungsintervall 23 erst nach der Stelle der sprunghaften Veränderung der Herzfrequenz HF beginnt. Dabei kann zusätzlich auch noch vorgesehen werden, dass eine gewisse Anzahl von Intervallen $RR_i$ 13 die in der Nähe der Stelle der sprunghaften Veränderung der Herzfrequenz HF liegen, von der weiteren Auswertung gänzlich ausgenommen werden. Das heißt, dass diese weder in die Folge der Intervalle $RR_i$ 13 des ersten Beobachtungsintervalls 22 noch in die entsprechende Folge des weiteren Beobachtungsintervall 23 aufgenommen werden. Zusätzlich kann auch noch vorgesehen sein, dass nicht nur der Beginn bzw. das Ende der Beobachtungsintervalle 22, 23 sondern dass auch die Länge bzw. die Anzahl der in den Beobachtungsintervallen 22, 23 berücksichtigten Intervalle $RR_i$ 13 in Abhängigkeit von während des Trainingsverlaufs auftretenden besonderen Ereignissen vorzugsweise automatisiert angepasst werden. Diese Maßnahmen der dynamischen Anpassung der Analysenrandbedingungen haben den Vorteil einer verbesserten Qualität bzw. erhöhten Zuverlässigkeit der Analyseergebnisse.

Gemäß einer alternativen Ausführungsvariante des Verfahrens ist vorgesehen, dass zur Analyse der jeweiligen Folgen von Intervallen $RR_i$ 13 eine lineare Trendbereinigung durchgeführt wird. Dazu wird unter Anwendung der Methode der kleinsten Abweichungsquadrate eine Regressionsgerade zu der jeweiligen Folge von Intervallen $RR_i$ 13 berechnet.

$$Y_i = a + b\,i;\ i = 1 \dots N$$

Die Folge der normierten Intervalle $rr_i$ 14 wird sodann dadurch gebildet, dass von den Intervallen $RR_i$ 13 zunächst die jeweiligen Werte $Y_i$ subtrahiert werden und der erhaltene Wert durch den Mittelwert $RR_{MW}$ der Intervalle $RR_i$ 13 dividiert wird.

$$rr_i = (RR_i - (a + b\,i)) / RR_{MW};\ i = 1 \dots N$$

Alternativ dazu kann die Folge der normierten Intervalle $rr_i$ 14 aber auch dadurch erzeugt werden, dass nicht durch den Mittelwert $RR_{MW}$, sondern durch den jeweiligen Wert $Y_i$ der entsprechenden Regressionsgerade dividiert wird.

$$rr_i = (RR_i - (a + b\,i)) / (a + b\,i);\ i = 1 \dots N$$

Durch die beschriebene lineare Trendbereinigung wird eine verbesserte Stationarität der normierten Intervalle $rr_i$ 14 erreicht, dazu Veränderungen der Herzfrequenz HF bzw. der Intervalle $RR_i$ 13 über das Beobachtungsintervall bzw. die Folge der Intervalle $RR_i$ hinweg ausgeglichen werden.

Gemäß einer weiteren alternativen Ausführungsvariante des Verfahrens erfolgt die Normierung bzw. die Abbildung der Intervalle $RR_i$ 13 auf die normierten Intervalle $rr_i$ 14 unter Verwendung eines Polynoms dritten Grades. Dazu wird unter Anwendung der Methode der Minimierung der Abweichungsquadrate zu der Folge der Intervalle $RR_i$ 13 eine polynomische Funktion dritten Grades berechnet. Das heißt, dass die Folge der Intervalle $RR_i$ 13 durch ein Polynom dritten Grades approximiert wird.

$$Y_i = a + b\,i + c\,i^2 + b\,i^3;\ i = 1 \ldots N$$

$$rr_i = (RR_i - Y_i) / Y_i;\ i = 1 \ldots N$$

Die in der zuletzt genannten Weise berechneten Folgen von normierten Intervallen $rr_i$ 14 werden sodann in gleicher Weise in einem Poincaré-Plot, wie in Fig. 5 dargestellt, eingetragen. Schließlich wird nach Berechnung der Hauptlängenachse $X_0$ 16 und der Hauptbreitenachse $Y_0$ 17 die Standardabweichung der orthogonalen Abstände 18 bezüglich der Hauptlängenachse $X_0$ 16 berechnet. Die zu den jeweils ausgewählten Folgen von Intervallen $RR_i$ 13 berechneten Standardabweichungen $SO_L$ und Mittelwerte $RR_{MW}$ der Intervalle $RR_i$ 13 bzw. die entsprechenden Herzfrequenzen HF werden schließlich zur Berechnung der Trainingsintensitätskurve 20 herangezogen. Infolge der gemäß diesem Ausführungsbeispiel verwendeten Normierung der Intervalle $RR_i$ 13 auf die normierten Intervalle $rr_i$ 14 ergibt sich ein noch deutlicher, hervortretender Anstieg der Standardabweichungen $SO_L$ im Bereich höherer Herzfrequenzen HF bzw. höherer Belastungen in der Trainingsintensitätskurve 20 (Fig. 6).

In wieder einer anderen alternativen Ausführungsvariante des Verfahrens wird zur Abbildung der Intervalle $RR_i$ 13 auf die normierten Intervalle $rr_i$ 14 eine Fourierreihen-Entwicklung verwendet. Intervalle $RR_i$ 13 werden durch eine Fourier-Entwicklung interpoliert, indem die Koeffizienten der Fourier-Entwicklung bestimmt werden. Zur Normierung der Intervalle $RR_i$ 13 wird schließlich ein trigonometrisches Polynom $F_i$ verwendet, das dadurch erhalten wird, dass die Fourier-Entwicklung nach einigen wenigen Gliedern abgebrochen wird. Vorzugsweise wird ein trigonometrisches Polynom bestehend aus den ersten drei Gliedern der Fourier-Entwicklung zur Berechnung der normierten Intervalle $rr_i$ 14 verwendet. Die Folge der Intervalle $RR_i$ 13 wird demnach durch die ersten drei Glieder der Fourier-Entwicklung approximiert.

$$rr_i = (RR_i - F_i) / F_i;\ i = 1 \ldots N$$

Die weitere Analyse der Folge der Intervalle $RR_i$ 13 bzw. der normierten Intervalle $rr_i$ 14 erfolgt wie vorstehend bereits ausgeführt worden ist, durch Eintrag der normierten Intervalle $rr_i$ 14 in einen Poincaré-Plot gemäß Fig. 5 und Berechnung der Standardabweichung $SO_L$ aus dem orthogonalen Abständen 18 der Punkte in dem Poincaré-Plot bezüglich der Hauptlängenachse $X_0$ 16. In weiterer Folge werden daraus Punkte der Trainingsintensitätskurve 20, wie in Fig. 6 dargestellt, berechnet und zur Beurteilung der momentanen Trainingsintensität der Person 2 herangezogen.

In einer weitem Ausführungsvariante des erfindungsgemäßen Verfahrens ist es auch möglich zur Normierung der Intervalle $RR_i$ 13 Reihenentwicklungen unter Verwendung so genannter Wavelets bzw. Wavelet-Transformationen zu verwenden. Von den dabei erhaltenen Reihenentwicklungen werden wiederum nur einige wenige Glieder zur Bildung eines Polynoms $W_i$ herangezogen. Vorzugsweise werden die ersten drei Glieder der Wavelet-Reihenentwicklung der Intervalle $RR_i$ 13 zur Normierung herangezogen.

$$rr_i = RR_i - W_i / W_i;\ i = 1 \ldots N$$

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Die Ausführungsbeispiele beschreiben mögliche Ausführungsvarianten des Verfahrens, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

Der Ordnung halber sei abschließend darauf hingewiesen, dass zum besseren Verständnis des Aufbaus der Vorrichtung 1 diese bzw. deren Bestandteile teilweise unmaßstäblich und/oder vergrößert und/oder verkleinert dargestellt wurden.

**Bezugszeichenaufstellung**

1 Vorrichtung
2 Person
3 Brustgurt
4 Sensor
5 Überwachungseinheit

6 Sender
7 Empfänger
8 Steuerung
9 Auswerteeinheit
10 Speicher

11 Bedieneinheit
12 Anzeige
13 Intervall $RR_i$
14 Intervall $rr_i$
15 Punktwolke

16 Hauptlängenachse $X_0$
17 Hauptbreitachse $Y_0$
18 Abstand
19 Abstand
20 Trainingsintensitätskurve

21 Referenzwert
22 Beobachtungsintervall
23 Beobachtungsintervall

**Patentansprüche**

1. Verfahren zur Bestimmung der Trainingsintensität einer Person (2), wobei die Herzfrequenzinformation der Person (2) gemessen wird, **dadurch gekennzeichnet, dass**

a) zeitliche Intervalle $RR_i$ (13) zwischen jeweils aufeinander folgenden Herzschlägen aufgezeichnet werden und
b) durch Festlegen einer Anzahl N von aufeinander folgenden Intervallen $RR_i$ (13) oder durch Festlegen eines Beobachtungsintervalls eine Folge von Intervallen $RR_i$ (13) ausgewählt wird und
c) aus der Folge der Intervalle $RR_i$ (13) durch eine mathematische Abbildung eine Folge von normierten Intervallen $rr_i$ (14) erzeugt wird und
d) aus jeweils zwei aufeinander folgenden normierten Intervallen $rr_i$ (14) und $rr_{i+1}$ Punkte in einem Poincaré-Plot der normierten Intervalle $rr_i$ (14) erzeugt werden und
e) zu den Punkten der Folge der normierten Intervalle $rr_i$ (14) im Poincaré-Plot eine Vertrauensellipse, insbesondere eine 95 % Vertrauensellipse, berechnet wird, wobei eine Hauptlängenachse $X_0$ (16) und eine Hauptbreitenachse $Y_0$ (17) bestimmt wird und
f) aus orthogonalen Abständen (18) der Punkte der Folge der normierten Intervalle $rr_i$ (14) bezüglich der Hauptlängenachse $X_0$ (16) eine Standardabweichung $SO_L$ berechnet wird und
g) ein Wertepaar bestehend aus einem Mittelwert $RR_{MW}$ der Folge der Intervalle $RR_i$ (13) und aus der Standardabweichung $SO_L$ abgespeichert wird und
h) weitere Folgen von Intervallen $RR_i$ (13) ausgewählt werden und für diese weiteren Folgen der Intervalle $RR_i$

(13) die Schritte c) bis g) ausgeführt werden und

i) aus in den Schritten c) bis h) bestimmten Wertepaaren von den Mittelwerten $RR_{MW}$ entsprechenden Herzfrequenzen HF (HF = 1 / $RR_{MW}$) und den Standardabweichungen $SO_L$ eine Trainingsintensitätskurve (20) der Person (2) erzeugt wird und

j) durch Vergleich der zuletzt bestimmten Herzfrequenz HF mit der Trainingsintensitätskurve (20) eine momentane, spezifische Trainingsintensität der Person (2) ermittelt wird.

2. Verfahren zur Bestimmung der Trainingsintensität einer Person (2), wobei die Herzfrequenzinformation der Person (2) gemessen wird, **dadurch gekennzeichnet, dass**

a) zeitliche Intervalle $RR_i$ (13) zwischen jeweils aufeinander folgenden Herzschlägen aufgezeichnet werden und

b) durch Festlegen einer Anzahl N von aufeinander folgenden Intervallen $RR_i$ (13) oder durch Festlegen eines Beobachtungsintervalls eine Folge von Intervallen $RR_i$ (13) ausgewählt wird und

c) aus der Folge der Intervalle $RR_i$ (13) durch eine mathematische Abbildung eine Folge von normierten Intervallen $rr_i$ (14) erzeugt wird und

d) aus jeweils zwei aufeinander folgenden normierten Intervallen $rr_i$ (14) und $rr_{i+1}$ Punkte in einem Poincaré-Plot der normierten Intervalle $rr_i$ (14) erzeugt werden und

e) zu den Punkten der Folge der normierten Intervalle $rr_i$ (14) im Poincare-Plot eine Vertrauensellipse, insbesondere eine 95 % Vertrauensellipse, berechnet wird, wobei eine Hauptlängenachse $X_0$ (16) und eine Hauptbreitenachse $Y_0$ (17) bestimmt wird und

f) aus orthogonalen Abständen (18) der Punkte der Folge der normierten Intervalle $rr_i$ (14) bezüglich der Hauptlängenachse $X_0$ (16) eine Standardabweichung $SO_L$ und aus orthogonalen Abständen (19) der Punkte der Folge der normierten Intervalle $rr_i$ (14) bezüglich der Hauptbreitenachse $Y_0$ (17) eine Standardabweichung $SO_W$ berechnet wird und

g) ein Wertepaar bestehend aus einem Mittelwert $RR_{MW}$ der Folge der Intervalle $RR_i$ (13) und aus einem aus der Standardabweichung $SO_L$ und aus der Standardabweichung $SO_W$ gebildeten Quotienten s = $SO_L$ / $SO_W$ abgespeichert wird und

h) weitere Folgen von Intervallen $RR_i$ (13) ausgewählt werden und für diese weiteren Folgen der Intervalle $RR_i$ (13) die Schritte c) bis g) ausgeführt werden und

i) aus in den Schritten c) bis h) bestimmten Wertepaaren von den Mittelwerten $RR_{MW}$ entsprechenden Herzfrequenzen HF (HF = 1 / $RR_{MW}$) und von aus den Standardabweichungen $SO_L$ und Standardabweichungen $SO_W$ gebildeten Quotienten s eine Trainingsintensitätskurve (20) der Person (2) erzeugt wird und

j) durch Vergleich der zuletzt bestimmten Herzfrequenz HF mit der Trainingsintensitätskurve (20) eine momentane, spezifische Trainingsintensität der Person (2) ermittelt wird.

3. Verfahren nach Anspruch 1 oder 2, **dadurch gekennzeichnet, dass** die zur Analyse vorgesehenen Folgen der Intervalle $RR_i$ (13) in Abhängigkeit von Ereignissen im Trainingsverlauf ausgewählt werden.

4. Verfahren nach Anspruch 3, **dadurch gekennzeichnet, dass** die Folgen der Intervalle $RR_i$ (13) so gewählt werden, dass Stellen einer sprunghaften Änderung der Herzfrequenz HF ausgenommen bzw. nicht enthalten sind.

5. Verfahren nach einem der vorhergehenden Ansprüche, **dadurch gekennzeichnet, dass** die zur Analyse vorgesehenen Folgen der Intervalle $RR_i$ (13) aus einander zeitlich überlappenden Beobachtungsintervallen ausgewählt werden.

6. Verfahren nach einem der vorhergehenden Ansprüche, **dadurch gekennzeichnet, dass** zur Identifikation und Elimination von Artefakten in der jeweiligen Folge der Intervalle $RR_i$ (13) jedem Intervall $RR_i$ (13) ein Referenzwert (21) zugeordnet wird, wobei der Referenzwert (21) durch einen Median der jeweils zumindest drei vorhergehenden und der zumindest drei nachfolgenden Intervalle $RR_i$ (13) gebildet wird, und, wenn das jeweilige Intervall $RR_i$ (13) um mehr als ein vorwählbarer Prozentsatz von dem ihm zugeordneten Referenzwert (21) abweicht, dieses Intervall $RR_i$ (13) durch seinen Referenzwert (21) ersetzt wird.

7. Verfahren nach Anspruch 6, **dadurch gekennzeichnet, dass** als Prozentsatz 30 % gewählt wird.

8. Verfahren nach Anspruch 6 oder 7, **dadurch gekennzeichnet, dass** Beobachtungsintervalle, die eine über eine Grenze von einem vorwählbaren Prozentsatz hinaus gehende Häufigkeit von Artefakten aufweisen, von einer weiteren Auswertung ausgeschlossen werden.

9. Verfahren nach Anspruch 8, **dadurch gekennzeichnet, dass** als Prozentsatz 5 % gewählt wird.

10. Verfahren nach einem der vorhergehenden Ansprüche, **dadurch gekennzeichnet, dass** zur Erzeugung der Folge der normierten Intervalle $rr_i$ (14) jedes Intervall $RR_i$ (13) durch einen Mittelwert $RR_{MW}$ der Folge der Intervalle $RR_i$ (13) dividiert wird, entsprechend der Formel

$$rr_i = RR_i / RR_{MW}; \; i = 1 \ldots N$$

11. Verfahren nach Anspruch 10, **dadurch gekennzeichnet, dass** zur Erzeugung der Folge der normierten Intervalle $rr_i$ (14) zusätzlich eine lineare Trendbereinigung mit einer Regressionsgeraden ($Y_i = a + b \, i$) zu der Folge der Intervalle $RR_i$ (13) durchgeführt wird, entsprechend der Formel

$$rr_i = (RR_i - (a + b \, i)) / RR_{MW}; \; i = 1 \ldots N$$

12. Verfahren nach einem der Ansprüche 1 bis 9, **dadurch gekennzeichnet, dass** zur Erzeugung der Folge der normierten Intervalle $rr_i$ (14) eine Approximation der Folge der Intervalle $RR_i$ (13) durch eine Regressionsgerade ($Y_i = a + b \, i$) verwendet wird, entsprechend der Formel

$$rr_i = (RR_i - (a + b \, i)) / (a + b \, i); \; i = 1 \ldots N$$

13. Verfahren nach einem der Ansprüche 1 bis 9, **dadurch gekennzeichnet, dass** zur Erzeugung der Folge der normierten Intervalle $rr_i$ (14) eine Approximation der Folge der Intervalle $RR_i$ (13) durch ein Polynom dritten Grades ($Y_i = a + b \, i + c \, i^2 + b \, i^3$) verwendet wird, entsprechend der Formel

$$rr_i = (RR_i - (a + b \, i + c \, i^2 + b \, i^3)) / (a + b \, i + c \, i^2 + b \, i^3); \; i = 1 \ldots N$$

14. Verfahren nach einem der Ansprüche 1 bis 9, **dadurch gekennzeichnet, dass** zur Erzeugung der Folge der normierten Intervalle $rr_i$ (14) eine Approximation der Folge der Intervalle $RR_i$ (13) durch trigonometrisches Polynom $F_i$ bestehend aus den ersten drei Gliedern einer Fourier-Entwicklung verwendet wird, entsprechend der Formel

$$rr_i = (RR_i - F_i) / F_i; \; i = 1 \ldots N$$

15. Verfahren nach einem der Ansprüche 1 bis 9, **dadurch gekennzeichnet, dass** zur Erzeugung der Folge der normierten Intervalle $rr_i$ (14) eine Approximation der Folge der Intervalle $RR_i$ (13) durch die ersten drei Glieder einer Wavelet-Reihenentwicklung $W_i$ verwendet wird, entsprechend der Formel

$$rr_i = (RR_i - W_i) / W_i; \; i = 1 \ldots N$$

16. Verfahren nach einem der vorhergehenden Ansprüche, **dadurch gekennzeichnet, dass** als Trainingsintensitätskurve (20) eine Approximation der Wertepaare, bestehend aus der Herzfrequenz HF und der Standardabweichung $SO_L$, oder eine Approximation der Wertepaare, bestehend aus der Herzfrequenz HF und dem Quotienten $s = SO_L / SO_W$, verwendet wird, entsprechend der Formel eines Polynoms, bevorzugt dritten Grades

$$SO_L(HF) = s_0 + s_1\,HF + s_2\,HF^2 + s_3\,HF^3$$

oder

$$s(HF) = u_0 + u_1\,HF + u_2\,HF^2 + u_3\,HF^3$$

17. Verfahren nach Anspruch 16, **dadurch gekennzeichnet, dass** die Trainingsintensitätskurve (20) in regelmäßigen zeitlichen Abständen neu berechnet wird.

18. Verfahren nach einem der vorhergehenden Ansprüche, **dadurch gekennzeichnet, dass** die Trainingsintensitätskurve (20) nach Abschluss des Trainings gemeinsam mit Daten, wie dem Namen der Person (2) und den Daten des durchgeführten Trainings, gespeichert wird.

19. Vorrichtung (1) zur Überwachung der Belastungsintensität einer Person (2) umfassend eine am Körper befestigbare Einheit mit einem Sensor (4) zur Aufzeichnung von Herzschlägen, **dadurch gekennzeichnet, dass** die Vorrichtung (1) eine Auswerteeinheit (9) mit Mitteln zur Durchführung eines Verfahrens nach einem der Ansprüche 1 bis 13 und eine Ausgabeeinheit zur Information der Person (2) über die ermittelte Belastungsintensität umfasst.

**Claims**

1. Method of determining the training intensity of a person (2), whereby the heart frequency data of the person (2) is measured, **characterised in that**

   a) time intervals $RR_i$ (13) between consecutive heartbeats are recorded and
   b) a sequence of intervals $RR_i$ (13) is selected by defining a number N of consecutive intervals $RR_i$ (13) or by defining an observation interval and
   c) a sequence of standardised intervals $rr_i$ (14) is produced from the sequence of intervals $RR_i$ (13) by mathematical modelling, and
   d) points are created in a Poincaré plot of the standardised intervals $rr_i$ (14) from two consecutive standardised intervals $rr_i$ (14) and $rr_{i+1}$, and
   e) a confidence ellipse, more particularly a 95% confidence ellipse, is calculated for the points of the sequence of standardised intervals $rr_i$ (14) in the Poincaré plot, and a principal horizontal axis $X_0$ (16) and a principal vertical axis $Y_0$ (17) is determined, and
   f) a standard deviation $SO_L$ is calculated from orthogonal distances (18) of the points of the sequence of standardised intervals $rr_i$ (14) from the principal horizontal axis $X_0$ (16), and
   g) a pair of values consisting of a mean value $RR_{MV}$ of the sequence of intervals $RR_i$ (13) and the standard deviation $SO_L$ is stored and
   h) further sequences of intervals $RR_i$ (13) are selected and steps c) to g) are carried out for these further sequences of intervals $RR_i$ (13), and
   i) a training intensity curve (20) is produced for the person (2) from the pairs of values of heart frequencies HF ($HF = 1/RR_{MV}$) corresponding to the mean values $RR_{MV}$ and standard deviations $SO_L$ determined in steps c) to h), and
   j) a current, specific training intensity is determined for the person (2) by comparing the most recently determined heart frequency HF with the training intensity curve (20).

2. Method of determining the training intensity of a person (2) whereby the heart frequency data of the person (2) is measured **characterised in that**

   a) time intervals $RR_i$ (13) between consecutive heartbeats are recorded, and
   b) a sequence of intervals $RR_i$ (13) is selected by defining a number N of consecutive intervals $RR_i$ (13) or by determining an observation interval, and
   c) a sequence of standardised intervals $rr_i$ (14) is generated from the sequence of intervals $RR_i$ (13) by mathematical modelling, and

d) points are generated in a Poincaré plot of the standardised intervals $rr_i$ (14) from two respective consecutive standardised intervals $rr_i$ (14) and $rr_{i+1}$, and

e) a confidence ellipse, more particularly a 95% confidence ellipse, is calculated for the points of the sequence of standardised intervals $rr_i$ (14) in the Poincaré plot, and a principal horizontal axis $X_0$ (16) and a principal vertical axis $Y_0$ (17) is determined, and

f) a standard deviation $SO_L$ is calculated from orthogonal distances (18) of the points of the sequence of standardised intervals $rr_i$ (14) from the principal horizontal axis $X_0$ (16) and a standard deviation $SO_W$ is calculated from orthogonal distances (19) of the points of the sequence of the standardised intervals $rr_i$ (14) from the principal vertical axis $Y_0$ (17), and

g) a pair of values consisting of a mean value $RR_{MV}$ of the sequence of intervals $RR_i$ (13), a quotient $s = SO_L / SO_W$ derived from the standard deviation $SO_L$ and standard deviation $SO_W$ is stored, and

h) further sequences of intervals $RR_i$ (13) are selected and steps c) to g) are carried out for these further sequences of intervals $RR_i$ (13), and

i) a training intensity curve (20) is produced for the person (2) from the pairs of values of heart frequencies HF ($HF = 1 / RR_{MV}$) corresponding to the mean values $RR_{MV}$ and quotient s derived from the standard deviations $SO_L$ and standard deviations $SO_W$ determined in steps c) to h), and

j) a current, specific training intensity is determined for the person (2) by comparing the most recently determined heart frequency HF with the training intensity curve (20).

3. Method as claimed in claim 1 or 2, **characterised in that** the sequences of intervals $RR_i$ (13) that will be used for analysis are selected depending on incidents during the course of training.

4. Method as claimed in claim 3, **characterised in that** the sequences of the intervals $RR_i$ (13) are selected in such a way that points of a sudden change in the heart frequency HF are excluded or not included.

5. Method as claimed in one of the preceding claims, **characterised in that** the sequences of the intervals $RR_i$ (13) for analysis are selected from observation intervals that overlap each other in time.

6. Method as claimed in one of the preceding claims, **characterised in that** in order to identify and eliminate artefacts in the relevant sequence of intervals $RR_i$ (13), a reference value (21) is assigned to each interval $RR_i$ (13), and the reference value (21) is formed by a median of the at least three preceding and the at least three subsequent intervals $RR_i$ (13), and if the relevant interval $R_i$ (13) deviates from its assigned reference value (21) by more than a pre-selectable percentage, this interval $RR_i$ (13) is replaced by its reference value (21).

7. Method as claimed in claim 6, **characterised in that** 30% is selected as the percentage.

8. Method as claimed in claim 6 or 7, **characterised in that** observation intervals which exhibit an artefact frequency beyond a limit of a pre-selectable percentage are excluded from further evaluation.

9. Method as claimed in claim 8 **characterised in that** 5 % is selected as the percentage.

10. Method as claimed in one of the preceding claims, **characterised in that** in order to generate the sequence of standardised intervals $rr_i$ (14), each interval $RR_i$ (13) is divided by a mean value $RR_{MV}$ of the sequence of intervals $RR_i$ (13) in accordance with the formula

$$rr_i = RR_i / RR_{MV}; \; i = 1 \dots N$$

11. Method as claimed in claim 10, **characterised in that** in order to generate the sequence of standardised intervals $rr_i$ (14), a linear trend elimination with a regression line ($Y_i = a + b\,i$) is also carried out for the sequence of intervals $RR_i$ (13) in accordance with the formula $rr_i = (RR_i - (a + b\,i)) / RR_{MW}; \; i = 1 \dots N$

12. Method as claimed in one of claims 1 to 9, **characterised in that** in order to generate the sequence of standardised intervals $rr_i$ (14), an approximation of the sequence of intervals $RR_i$ (13) through a regression line ($Y_i = a + b\,i$) is used in accordance with the formula

$$rr_i = (RR_i - (a + b\ i)) / (a + b\ i);\ i = 1\ ...\ N$$

13. Method as claimed in one of claims 1 to 9, **characterised in that** in order to generate the sequence of standardised intervals $rr_i$ (14), an approximation of the sequence of intervals $RR_i$ (13) by a third degree polynomial ($Y_i = a + b\ i + c\ i^2 + b\ i^3$) is used in accordance with the formula $rr_i = (RR_i - (a+b\ i+c\ i^2 + b\ i^3)) / (a + b\ i + c\ i^2 + b\ i^3);\ i = 1... N$

14. Method as claimed in one of claims 1 to 9, **characterised in that** in order to generate the sequence of standardised intervals $rr_i$ (14), an approximation of the sequence of intervals $RR_i$ (13) by trigonometric polynomial $F_i$ consisting of the first three components of a Fourier transform is used in accordance with the formula $rr_i = (RR_i - F_i) / F_i;\ i = 1\ ...\ N$

15. Method as claimed in one of claims 1 to 9, **characterised in that** in order to generate the sequence of standardised intervals $rr_i$ (14), an approximation of the sequence of intervals $RR_i$ (13) by the first three components of a wavelet series development $W_i$ is used in accordance with the formula $rr_i = (RR_i - W_i) / W_i;\ i = 1\ ...\ N$

16. Method as claimed in one of the preceding claims **characterised in that** as the training intensity curve (20), an approximation of the pairs of values consisting of the heart frequency HF and the standard deviation $SO_L$, or an approximation of the pairs of values consisting of the heart frequency HF and the quotient $s = SO_L / SO_W$ is used in accordance with the formula of a, preferably third-degree, polynomial, $SO_L(HF) = s_0 + s_1 HF + s_2 HF^2 + s_3 HF^3$ or $s(HF) = u_0 + u_1 HF + u_2 HF^2 + u_3 HF^3$

17. Method as claimed in claim 16, **characterised in that** the training intensity curve (20) is recalculated at regular time intervals.

18. Method as claimed in one of the preceding claims, **characterised in that** the training intensity curve (20) is saved on completion of the training along with data such as the name of the person (2) and the dates on which the training was carried out.

19. Device (1) for monitoring the stress intensity of a person (2) comprising a unit attachable to the body with a sensor (4) for recording heartbeats, **characterised in that** the device (1) comprises an evaluation unit (9) with means for implementing a method as claimed in one of claims 1 to 13 and an output unit for informing the person (2) of the determined stress intensity.

**Revendications**

1. Procédé pour déterminer l'intensité d'entraînement d'une personne (2), dans lequel l'information sur la fréquence cardiaque de la personne (2) est mesurée, **caractérisé en ce que**

   a) des intervalles de temps $RR_i$ (13) entre des battements cardiaques consécutifs sont enregistrés et
   b) une séquence d'intervalles $RR_i$ (13) est sélectionnée par fixation d'un nombre N d'intervalles $RR_i$ (13) successifs ou par fixation d'un intervalle d'observation et
   c) une succession d'intervalles normalisés $rr_i$ (14) est générée à partir de la séquence des intervalles $RR_i$ (13) par une représentation mathématique et
   d) des points sont générés à partir de respectivement deux intervalles $rr_i$ (14) et $rr_{i+1}$ normalisés et consécutifs dans un tracé de Poincaré des intervalles $rr_i$ (14) normalisés
   e) une ellipse de confiance, en particulier une ellipse de confiance de 95 %, est calculée en ce qui concerne les points de la séquence des intervalles $rr_i$ (14) normalisés dans le tracé de Poincaré, un axe de longueur principale $X_0$ (16) et un axe de largueur principale $Y_0$ (17) étant déterminés et
   f) un écart standard $SO_L$ est calculé à partir d'espacements (18) orthogonaux des points de la séquence des intervalles $rr_i$ (14) normalisés par rapport à l'axe de longueur principale $X_0$ (16) et
   g) un couple de valeurs constitué d'une valeur moyenne $RR_{MW}$ de la séquence des intervalles $RR_i$ (13) et de l'écart standard $SO_L$ est mémorisé et
   h) d'autres séquences d'intervalles $RR_i$ (13) sont sélectionnées et les étapes c) à g) sont exécutées pour ces autres séquences des intervalles $RR_i$ (13) et
   i) une courbe d'intensité d'entraînement (20) de la personne (2) est générée à partir de couples de valeurs, déterminés aux étapes c) à h), des fréquences cardiaques HF (HF = $1/RR_{MW}$) correspondant à des valeurs moyennes $RR_{MW}$ et des écarts standard $SO_L$ et

j) une intensité d'entraînement momentanée spécifique de la personne (2) est déterminée par comparaison de la dernière fréquence cardiaque HF déterminée avec la courbe d'intensité d'entraînement (20).

2. Procédé pour déterminer l'intensité d'entraînement d'une personne (2), dans lequel l'information sur la fréquence cardiaque de la personne (2) est mesurée, **caractérisé en ce que**

   a) des intervalles de temps $RR_i$ (13) entre des battements cardiaques consécutifs sont enregistrés et

   b) une séquence d'intervalles $RR_i$ (13) est sélectionnée par fixation d'un nombre N d'intervalles successifs $RR_i$ (13) ou par fixation d'un intervalle d'observation et

   c) une séquence d'intervalles normalisés $rr_i$ (14) est générée à partir de la séquence des intervalles $RR_i$ (13) par une représentation mathématique et

   d) des points sont générés à partir de respectivement deux intervalles $rr_i$ (14) et $rr_{i+1}$ normalisés consécutifs dans un tracé de Poincaré des intervalles $rr_i$ (14) normalisés et

   e) une ellipse de confiance, en particulier une ellipse de confiance de 95 %, est calculée en ce qui concerne les points de la séquence des intervalles $rr_i$ (14) normalisés dans le tracé de Poincaré, un axe de longueur principale $X_0$ (16) et un axe de largueur principale $Y_0$ (17) étant déterminés et

   f) un écart standard $STO_L$ est calculé à partir d'espacements orthogonaux (18) des points de la séquence des intervalles normalisés $rr_i$ (14) par rapport à l'axe de longueur principale $X_0$ (16) et un écart standard $SO_W$ est calculé à partir d'espacements orthogonaux (19) des points de la séquence des intervalles normalisés $rr_i$ (14) par rapport à l'axe de largeur principale $Y_0$ (17) et

   g) un couple de valeurs constitué d'une valeur moyenne $RR_{MW}$ de la séquence des intervalles $RR_i$ (13) et d'un quotient $s = SO_L/SO_W$ formé de l'écart standard $SO_L$ et de l'écart standard $SO_W$ est mémorisé et

   h) d'autres séquences d'intervalles $RR_i$ (13) sont sélectionnées et les étapes c) à g) sont exécutées pour ces autres séquences des intervalles $RR_i$ (13) et

   i) une courbe d'intensité d'entraînement (20) de la personne (2) est générée à partir des couples de valeurs déterminés aux étapes c) à h) des fréquences cardiaques HF (HF = /$RR_{MW}$) correspondant aux valeurs moyennes $RR_{MW}$ et de quotients $s$ formés à partir des écarts standard $SO_L$ et des écarts standard $SO_W$ et

   j) une intensité d'entraînement momentanée spécifique de la personne (2) est déterminée par comparaison de la dernière fréquence cardiaque déterminée HF avec la courbe d'intensité d'entraînement (20).

3. Procédé selon la revendication 1 ou 2, **caractérisé en ce que** les séquences, prévues pour l'analyse, des intervalles $RR_i$ (13) sont sélectionnées en fonction d'événements dans la courbe d'entraînement.

4. Procédé selon la revendication 3, **caractérisé en ce que** les séquences des intervalles $RR_i$ (13) sont choisies de telle sorte que des emplacements d'une brusque modification de la fréquence cardiaque HF font exception ou ne sont pas inclus.

5. Procédé selon l'une quelconque des revendications précédentes, **caractérisé en ce que** les séquences, prévues pour l'analyse, des intervalles $RR_i$(13) sont sélectionnées à partir d'intervalles d'observation se chevauchant dans le temps.

6. Procédé selon l'une quelconque des revendications précédentes, **caractérisé en ce que**, pour l'identification et l'élimination d'artéfacts dans la séquence respective des intervalles $RR_i$ (13), une valeur de référence (21) est attribuée à chaque intervalle $RR_i$ (13), la valeur de référence (21) étant formée par une médiane des respectivement au moins trois intervalles $RR_i$ (13) précédents et des au moins trois intervalles $RR_i$(13) suivants, et, si l'intervalle respectif $RR_i$ (13) s'écarte de plus d'un pourcentage pouvant être présélectionné de la valeur de référence (21) qui lui est attribuée, cet intervalle $RR_i$ (13) est remplacé par sa valeur de référence (21).

7. Procédé selon la revendication 6, **caractérisé en ce que** 30 % est choisi comme pourcentage.

8. Procédé selon la revendication 6 ou 7, **caractérisé en ce que** des intervalles d'observation, qui présentent une fréquence d'artéfacts allant au-delà d'une limite d'un pourcentage pouvant être présélectionné, sont exclus d'une autre analyse.

9. Procédé selon la revendication 8, **caractérisé en ce que** 5 % est choisi comme pourcentage.

10. Procédé selon l'une quelconque des revendications précédentes, **caractérisé en ce que**, pour générer la séquence

des intervalles normalisés $rr_i$ (14), chaque intervalle $RR_i$ (13) est divisé par une valeur moyenne $RR_{MW}$ de la séquence des intervalles $RR_i$ (13), selon la formule suivante

$$rr_i = RR_i/RR_{MW}; \ i = 1... \ N.$$

**11.** Procédé selon la revendication 10, **caractérisé en ce que**, pour générer la séquence des intervalles normalisés $rr_i$ (14), on effectue en supplément une correction de tendance linéaire avec une droite de régression ($Y_i = a + b \ i$) pour la séquence des intervalles $RR_i$ (13), selon la formule suivante

$$rr_i = (RR_i - (a + b \ i))/RR_{MW}; \ i = 1... \ N.$$

**12.** Procédé selon l'une quelconque des revendications 1 à 9, **caractérisé en ce que**, pour générer la séquence des intervalles normalisés $rr_i$ (14), on utilise une approximation de la séquence des intervalles $RR_i$ (13) par une droite de régression ($Y_i = a + b \ i$), selon la formule suivante

$$rr_i = (RR_i - (a + b \ i))/(a + b \ i); \ i = 1... \ N.$$

**13.** Procédé selon l'une quelconque des revendications 1 à 9, **caractérisé en ce que**, pour générer la séquence des intervalles normalisés $rr_i$ (14), on utilise une approximation de la séquence des intervalles $RR_i$ (13) par un polynôme du troisième degré ($Y_i = a + b \ i + c \ i^2 + b \ i^3$), selon la formule suivante

$$rr_i = (RR_i - (a + b \ i + c \ i^2 + b \ i^3))/(a + b \ i + c \ i^2 + b \ i^3); \ i = 1... \ N.$$

**14.** Procédé selon l'une quelconque des revendications 1 à 9, **caractérisé en ce que**, pour générer la séquence des intervalles normalisés $rr_i$ (14), on utilise une approximation de la séquence des intervalles $RR_i$ (13) par un polynôme trigonométrique $F_i$ constitué des trois premiers membres d'un développement de Fourier, selon la formule suivante

$$rr_i = (RR_i - F_i)/F_i; \ i = 1... \ N.$$

**15.** Procédé selon l'une quelconque des revendications 1 à 9, **caractérisé en ce que**, pour générer la séquence des intervalles normalisés $rr_i$ (14), on utilise une approximation de la séquence des intervalles $RR_i$ (13) par les trois premiers membres d'un développement de série d'ondelettes $W_i$, selon la formule suivante

$$rr_i = (RR_i - W_i)/W_i; \ i = 1... \ N.$$

**16.** Procédé selon l'une quelconque des revendications précédentes, **caractérisé en ce que**, comme courbe d'intensité d'entraînement (20), on utilise une approximation des couples de valeurs, constitués de la fréquence cardiaque HF et de l'écart standard $SO_L$, ou une approximation des couples de valeurs, constitués de la fréquence cardiaque HF et du quotient $s = SO_L/SO_W$, selon la formule d'un polynôme, de préférence du troisième degré

$$SO_L(HF) = s_0 + s_1 \ HF + s_2 \ HF^2 + s_3 \ HF^3$$

ou

$$S(HF) = u_0 + u_1 \ HF + u_2 \ HF^2 + u_3 \ HF^3.$$

**17.** Procédé selon la revendication 16, **caractérisé en ce que** la courbe d'intensité d'entraînement (20) est recalculée à des intervalles de temps réguliers.

**18.** Procédé selon l'une quelconque des revendications précédentes, **caractérisé en ce que** la courbe d'intensité d'entraînement (20) est mémorisée après la fin de l'entraînement conjointement avec des données, telles que le nom de la personne (2) et les données de l'entraînement effectué.

**19.** Dispositif (1) pour contrôler l'intensité de charge d'une personne (2) comprenant une unité pouvant être fixée sur le corps avec un capteur (4) pour l'enregistrement de battements cardiaques, **caractérisé en ce que** le dispositif (1) comprend une unité d'analyse (9) avec des moyens pour la mise en oeuvre d'un procédé selon l'une quelconque des revendications 1 à 13 et une unité d'édition pour l'information de la personne (2) sur l'intensité de charge déterminée.

# Fig.1

# Fig.2

**Fig.3**

**Fig.4**

**Fig.5**

**Fig.6**

## Fig.7

$$s=\frac{SO_L}{SO_W}$$

HF

20

## Fig.8

$RR_i$

i

13
13 13
21
13
13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1127543 B1